# EUROPEAN PATENT APPLICATION

(11) **EP 2 025 364 A1**
(43) Date of publication of application: **18.02.2009**
(21) Application number: 07114264.0
(22) Date of filing: 13.08.2007
(51) Int. Cl.: A61Q 1/02, A61Q 1/06, A61Q 1/10, A61Q 5/02, A61Q 5/12, A61Q 19/00, A61K 8/11, A61K 8/25

(54) **Compositions comprising dye-loaded particles**

(71) Applicant: Procter & Gamble International Operations SA., 1213 Petit-Lancy 1 (CH)
(72) Inventor: Alberius, Peter, Carl, Anders, 183 51, Taby (SE); Corkery, Robert, William, 11727, Stockholm (SE); Stephens, Alison, Fiona, Cookham Maidenhead, Berkshire SL6 9LA (GB)
(74) Representative: Wilding, Richard Alan

(57) **Abstract**

According to the invention, a cosmetic composition is provided comprising:
(a) amorphous particles, each amorphous particle comprising a homogeneous distribution of one or more dyes encapsulated by an amorphous, siliceous encapsulating agent, wherein the amorphous particle comprises from 3% to 20%, preferably 5% to 15%, more preferably 8% to 12% dye, by weight of the particle;
(b) a cosmetically acceptable carrier.

## Description

### FIELD OF THE INVENTION

The present invention relates to cosmetic formulations comprising encapsulated dyes.

### BACKGROUND TO THE INVENTION

The provision of dyes is very important in the cosmetic field, in which altering skin and hair colour for aesthetic purposes can be desirable.

Currently, cosmetic compositions typically comprise pigments, which do not provide the range of colour desired by the skilled cosmetic formulator. Organic pigments might provide a solution to this problem, but the use of such materials in a cosmetic context is limited for reasons, such as safety and other disadvantageous effects, such as skin staining. One way to mitigate the problems associated with organic dyes could be to incorporate them in other materials.

Particles incorporating dyes for use in the present field are described in WO-A-2004/081222. This document describes a process for manufacturing encapsulated dyes using the well-known sol-gel methodology, which is an emulsion technique resulting in a core/shell structure (a core of dye surrounded by a shell of a material, such as silica). Further examples of particles of the art are found in US-A-2005/0276774 and US-A-2005/0265938, which describe the production of such particles by dispersive techniques and micelle formation respectively. However, particles known in the art often exhibit leakage of the dyes from the particles into which they have been incorporated, which is clearly undesirable - the same safety and skin staining concerns arise as in the case of unencapsulated organic dyes.

Accordingly, there is a need for cosmetic compositions comprising new dye particles, which can be reliably and effectively incorporated into cosmetic compositions whilst retaining all of the benefits of dyes already known in the art, i.e. good chemical and physical stability, colour fastness and tint strength as well as an acceptable environmental profile, but which at the same time show negligible to no leakage of the dyes from the particles over their lifetime.

### SUMMARY OF THE INVENTION

According to the invention, a cosmetic composition is provided comprising:
(a) amorphous particles, each amorphous particle comprising a homogeneous distribution of one or more dyes encapsulated by an amorphous, siliceous encapsulating agent, wherein the amorphous particle comprises from 3% to 20%, preferably 5% to 15%, more preferably 8% to 12% dye, by weight of the particle;
(b) a cosmetically acceptable carrier.

### BRIEF DESCRIPTION OF THE DRAWINGS

The invention is described with reference to the following drawings, in which:
Figure 1 is a schematic representation of an apparatus suitable for the production of particles of the present invention.
Figure 2 is a schematic representation of the nozzle used in the apparatus shown in Fig. 2.
Figure 3 is the calibration for ion-exchanged Tartrazine in water at 423 nm.
Figure 4 shows the tartrazine leakage obtained in Example 4B.

### DETAILED DESCRIPTION OF THE INVENTION

The dimensions and values disclosed herein are not to be understood to be strictly limited to the exact numerical values recited. Instead, unless otherwise stated, each dimension is intended to mean both the recited value and a functionally equivalent range surrounding that value. For example, a dimension disclosed as "40nm" is intended to mean "about 40nm".

In the context of the present invention, the term "encapsulation" is understood to mean that the dye is fully surrounded or encased by an encapsulating agent and, thus, held securely within the particle. Leakage of less than 5 weight %, preferably less than 2 weight%, more preferably less than 1 weight% of the total amount of dye incorporated into the particle is achieved, as determined by the methodology described herein in Example 4.

Both the present particles and the encapsulating agent comprised within the particles of the present invention are amorphous. In the context of the invention, the term "amorphous" means that there is no long range crystallographic order in one, two or three dimensions at lengths from 0.1- 50nm, as determined in the following way by a combination of powder x-ray diffraction (XRD) on bulk samples and transmission electron microscopy (TEM) of representative portions of the same bulk sample:
(a) The presence of a broad peak in the x-ray diffractogram centered between 2 theta angles corresponding to d-spacings of 0.37-0.42 nm, with full width half-maximum (FWHM) of between 5-10 degrees 2 theta;
(b) The lack of sharp powder x-ray diffraction peaks corresponding to spacings of crystallographic planes separated by 0.37-0.42nm;
(c) The lack of mesocrystalline order (where respective highest order Bragg peaks fall in the range 2-50nm - typical of ordered mesostructured materials), as determined by TEM imaging of samples prepared by microtoming;
(d) The lack of a multiplicity of sharp peaks in the range of two theta angles corresponding to d-spacings to 0.1-50nm.

This definition excludes ordered mesoporous materials with pore sizes from 2-50nm arranged with translational crystallographic order, such as MCM-41, MCM-48 and SBA-15.

In the context of the present invention, the term "siliceous" takes its normal meaning known in the art. More specifically, a siliceous material is one of, relating to or containing silica or a silicate. Preferably, the encapsulating agent is silica *per se.* Optionally, however, a proportion of the silicon within the amorphous silica structure may be substituted with other elements such as boron, lead, titanium, tin, zirconium and/or aluminium. This substitution of the silica framework may be useful in adjusting the properties of the silica-based particles depending upon their specific applications. For example, addition of boron, lead, tin, zirconium and/or aluminium may result in a different refractive index.

In addition, depending upon the desired applications and/or effects of the particles, it may be desirable for them to additionally comprise one or more inorganic particles, such that they comprise not only the siliceous encapsulating agent and one or more dyes, but also discrete inorganic, preferably refractory, particles such as titanium dioxide, zinc oxide, aluminium oxide and mixtures thereof, within their structure. For instance, titanium oxide and zinc oxide may provide additional sunscreen benefits. Such additional particles preferably have a mass average particle size of less than about 1 µm, preferably less than 100nm.

The dye molecules are typically present within the particle in more than one area or "pocket". This beneficially maximises the dye to particle volume or weight ratio and, thus, maximises the amount of dye ultimately included in the desired end compositions, for example the cosmetic, health, beauty or detergent products or ink compositions, whilst minimising the overall proportion of dye particles within such compositions. Irrespective of the number of areas or "pockets" of dye within the particle, each is fully surrounded or encapsulated by the encapsulating agent and, thus, held securely therein. Therefore, within the structure of the particles themselves, the encapsulating agent may be thought of as a continuous phase or matrix, whereas the dye may be thought of as comprised within a discontinuous phase. It follows that an "encapsulating agent" is an agent, which may be used to achieve this effect.

In this respect, the encapsulating agent may also be considered to be polymeric in nature because it will tend to possess crosslinking within its structure. It is preferred that the particles of the invention have as high a degree of crosslinking as possible, such that the dye is most effectively retained within the resulting particle and cannot leach therefrom. The degree of crosslinking may be observed using standard techniques such as Fourier transform infrared spectroscopy (FTIR) or solid state nuclear magnetic resonance spectroscopy (solid state NMR). Ideally and as previously mentioned, leakage of less than 5 weight %, preferably less than 2 weight%, more preferably less than 1 weight% of the total amount of dye incorporated into the particle is achieved, as determined by the methodology described herein in Example 4.

Additionally, the particles of the invention comprise a homogeneous distribution of the one or more dyes within the encapsulating agent. In the context of the present invention, this "homogeneous distribution" of the dye is understood to mean that the dye is homogeneously dispersed throughout the particle on a "molecular level". This means that the dye, typically present in one or more areas or "pockets", is not visible or discernible *via* microscopic techniques down to a range or magnification of 2nm. In other words, the particles of the invention appear as a homogeneous or single material at this level of microscopic magnification.

Turning now to the dyes included in the particles of the invention, a wide variety of dyes is suitable for this purpose. In the context of the present invention, the term "dye" refers to any dye or colorant, which is desired to be introduced into a particle and indefinitely retained within that particle. Examples of dyes which may be comprised within particles of the present invention include, but are not limited to, dyes or colorants conventionally used in the end application(s) of choice. For example, the suitability of dyes for use in applications such as cosmetic, health, personal care and detergent compositions is governed by organisations such as the Food and Drug Administration (FDA) in the USA and equivalent bodies in other countries. Typically, dyes suitable for use in the present invention may be cationic, anionic, neutral, amphoteric, zwitterionic or amphiphilic, with cationic dyes being preferred, as the positive charge on the dye molecule interacts with residual negative charge on the siliceous encapsulating agent to promote retention of the dye within the encapsulate. The dyes are typically selected from conventionally-known dye types such as natural dyes, ie. those derived from natural sources or synthetic equivalents thereof, azo dyes, indigoid dyes, triaryl-methane dyes, anthraquinone dyes, xanthine (xanthene) dyes, nitrosulphonate dyes, pyrene dyes, thiophene dyes, quinoline dyes and derivatives, lakes, composites or mixtures thereof, in particular those which have been approved for use by the FDA. Examples of suitable dyes are provided in the following tables (1 and 2), with their general dye types shown in brackets.

| **Table 1: Colour Additives batch-certified by the FDA** | | |
|---|---|---|
| **Standard Name** | **Chemical Structure** | **Colour Index Number (CI)** |
| FD&C Black No. 2 | Carbon black | 77266 |
| FD&C Orange No. 4 (monoazo) | 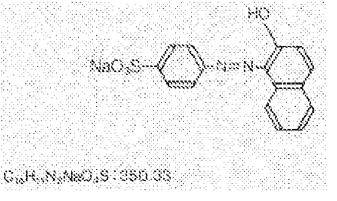 | 15510 |
| FD&C Orange No. 5 (xanthene-based) | 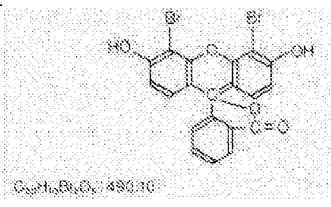 | 45370 |
| FD&C Orange No. 10 (xanthene-based) | 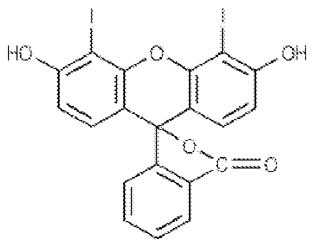 | 45425 |
| FD&C Orange No. 11 (Sodium salt of Orange No. 10; xanthene-based) | 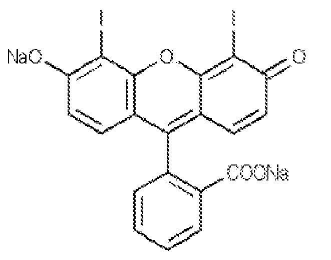 | 45425 |
| FD&C Blue No. 1 (triarylmethane; "Erioglaucine") | 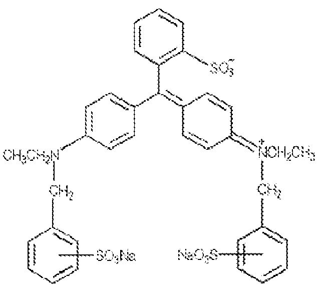 | 42090 |
| FD&C Blue No. 4 (triarylmethane) | 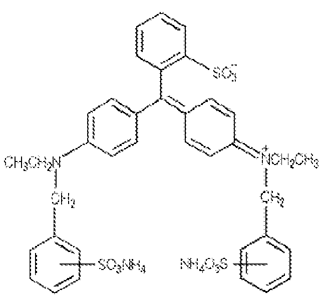 | 42090 |
| FD&C Brown No. 1 (diazo) | 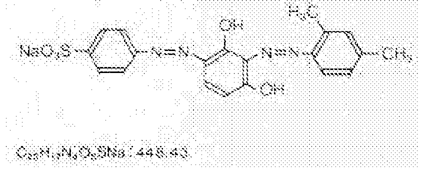 | 20170 |
| FD&C Violet No. 2 (anthracene dione-based; ie. anthraquinone based) | 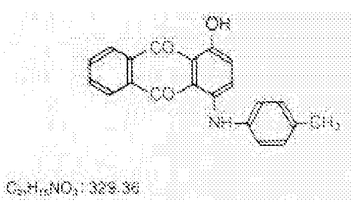 | 60725 |
| Ext. D&C Violet No. 2 (anthracene-based) | 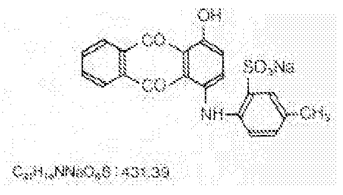 | 60730 |
| FD&C Green No. 3 (triarylmethane) | 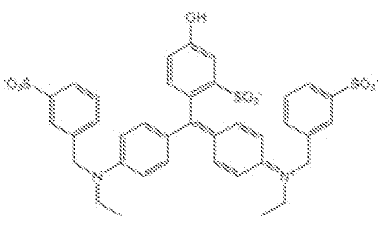 | 42053 |
| FD&C Green No. 5 (anthracene-based) | 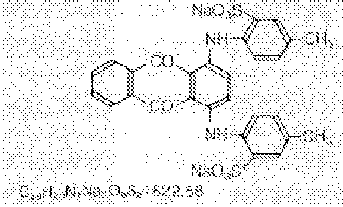 | 61570 |
| FD&C Green No. 6 (anthracene-based) | 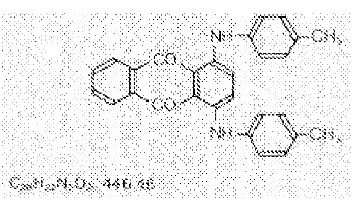 | 61565 |
| FD&C Green No. 8 (pyrene-based) | 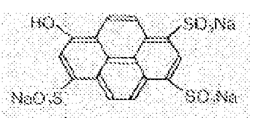 | 59040 |
| | | |
| FD&C Red No. 2 (monoazo; "Amaranth") | 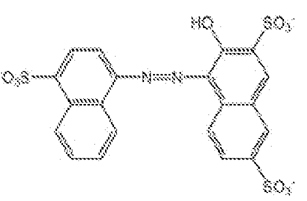 | 16185 |
| FD&C Red No. 4 (monoazo) | 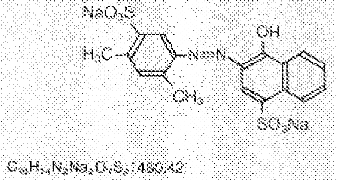 | 14700 |
| FD&C Red No. 6 (monoazo) | 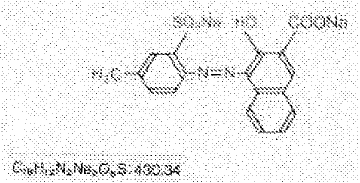 | 15850 |
| FD&C Red No. 7 (monoazo) | 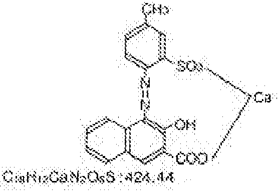 | 15850 |
| FD&C Red No. 17 (diazo) | 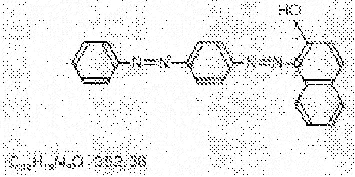 | 26100 |
| FD&C Red No. 21 (xanthene-based) | 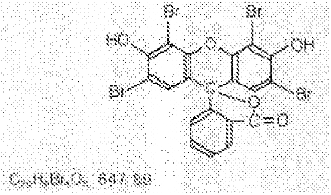 | 45380 |
| | | |
| FD&C Red No. 22 (xanthene-based) | 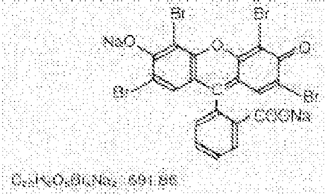 | 45380 |
| FD&C Red No. 27 (xanthene-based) | 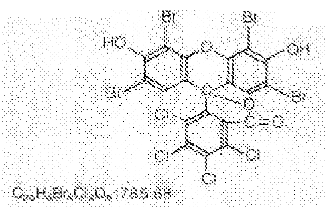 | 45410 |
| FD&C Red No. 28 (xanthene-based) | 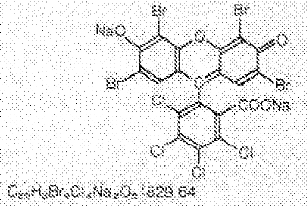 | 45410 |
| FD&C Red No. 30 (thiophene-based) | 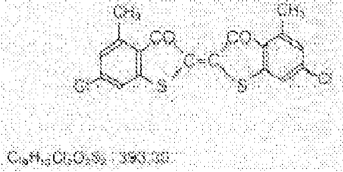 | 73360 |
| FD&C Red No. 31 (monoazo) | 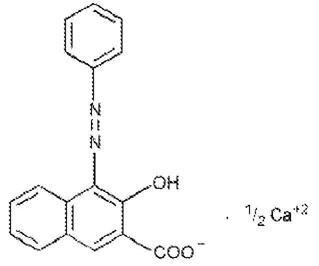 | 15800 |
| FD&C Red No. 33 (monoazo) | 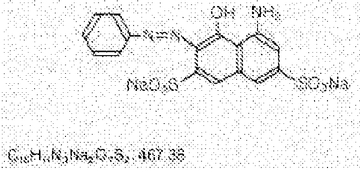 | 17200 |
| FD&C Red No. 34 (monoazo) | 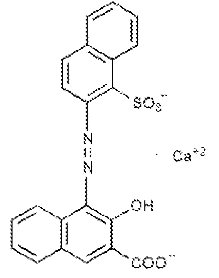 | 15880 |
| FD&C Red No. 40 (monoazo) | 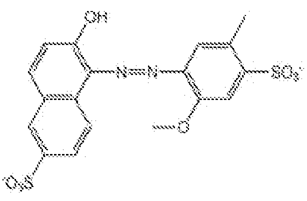 | 16035 |
| FD&C Yellow No. 5 (monoazo; "Tartrazine") | 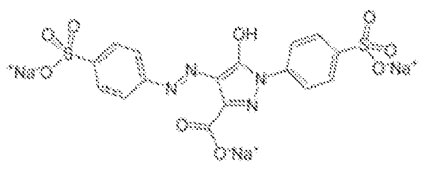 | 19140 |
| FD&C Yellow No. 6 (monoazo) | 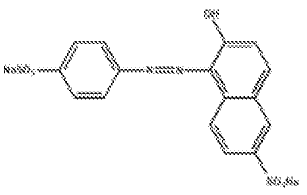 | 15985 |
| FD&C Yellow No. 7 (xanthene-based) | 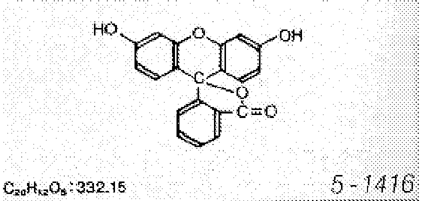 | 45350 |
| Ext. D&C Yellow No.7 (dinitroarylsulphonate) | 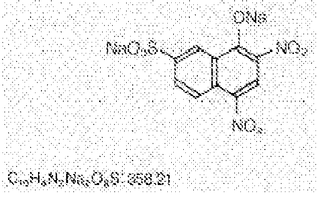 | 10316 |
| FD&C Yellow No. 8 (xanthene-based) | 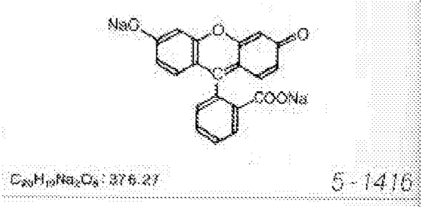 | 45350 |
| FD&C Yellow No. 10 (quinoline-based) | 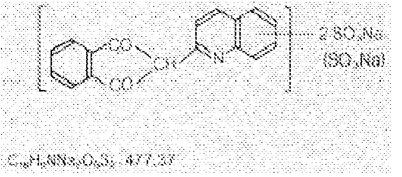 | 47005 |
| FD&C Yellow No. 11 (quinoline-based) | 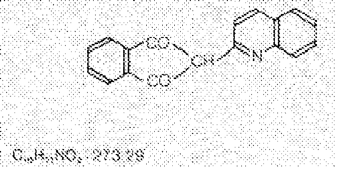 | 47000 |

| **Table 2: Natural Colour Additives which are Exempt from Batch Certification by the FDA** | | |
|---|---|---|
| **Name** | **Structure** | **CI** |
| Caramel | Not applicable (n//a) | |
| Cochineal | 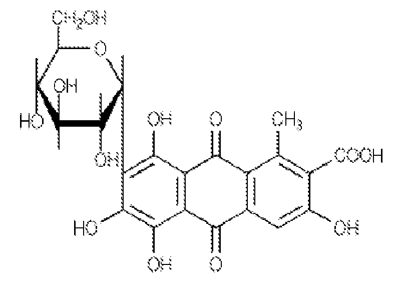 | 75470 |
| Beta carotene | 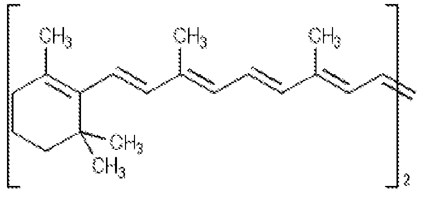 | 40800 or 75130 |
| Guanine | 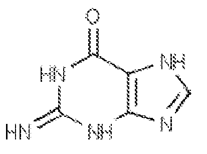 | 75170 |
| Henna | n/a | n/a |

The dye may be used in an unadulterated form or it may be adapted to improve its suitability to the present process. In particular, the effectiveness of some dyes containing anionic groups and a mono-valent alkali metal counter-ion, such as sodium, may be improved by ion-exchanging the metal ion with a mono-valent organic counter-ion such as ammonium or tetra-methyl ammonium.

Particularly preferred colorants or dyes include xanthene, triarylmethane, anthracene, and monoazo dyes.

The amount of dye included in the particles of the invention can be varied in accordance with the desired applications or effects of the particles, and may also depend upon the type(s) of dye chosen to be included within the particles. Within the particles of the invention, the proportion of dye(s) is from 3% to 20%, by weight of the particle. Preferably, the proportion of dye is in the range from 5% to 15%, and more preferably from 8 to 12%, by weight of the particle. These ranges have been found to equate exactly to the percentages of the starting materials used to make the particles.

The particles of the invention have a volume average particle size which renders them useful in the end application of choice. For instance, if the particles are to be used in cosmetic or beauty formulations, it is desirable that they are not discernible to the naked eye. Thus, such particles will typically have an average size of less that about 70 µm. However, if the particles are destined for used in detergent or other formulations, they may have greater sizes, for example. For cosmetic applications, the average particle size is generally in the range of greater than 0 to 10 µm, preferably in the range of greater than 0 to 5 µm, more preferably from greater than 0 to less than 1 µm and even more preferably, is from 10nm to less than 1µm. The average particle size of the particles is measured using standard techniques of the art, such as light scattering *via* use of a Malvern Sizer 2000 apparatus or by scanning electron microscopy (SEM).

The particles of the invention may have any shape appropriate to the end use in question. Preferably, the particles according to the invention are spherical because such particles may have more predictable qualities, such as optical and rheological properties. Within a cosmetic application, spherical particles may also provide improved skin feel, since they may act as a lubricant by providing a ball-bearing type effect.

The particles of the invention achieve effective retention of dyes therein by an amorphous, siliceous encapsulating agent. The invention provides particles which possess good chemical and physical stability, colour fastness and tint strength as well as an acceptable environmental profile. A corollary of the low dye leakage is that the surface of the silica encapsulates according to the present invention has similar properties to silica per se, regardless of the dye(s) incorporated therein. Thus, the particles may be reliably and effectively incorporated into compositions for use in a wide variety of applications to provide colorants, which show negligible to no leakage from the compositions into which they are incorporated and which, at the same time, provide more robust coloration to the compositions than colorants of the art. Because of this, the particles of the invention may be effectively used to provide previously unattainable dye combinations, as the individual dyes are securely held in the inventive particles.

In addition, the dye particles of the invention may be formulated into bulk colorant compositions for convenient "drop-in" use in the desired end compositions. This is particularly advantageous as end compositions currently formulated typically require specific, tailored formulation of all their individual components, including their colorants, in order to provide the correctly formulated end composition. Thus, use of colorant particles of the present invention obviates the need for this repetitive, time-consuming and, therefore, uneconomic "custom" formulation by enabling the formulation of bulk end-product compositions which may then be coloured as desired using bulk colorant compositions comprising pre-determined proportions of dye particles made by the present invention.

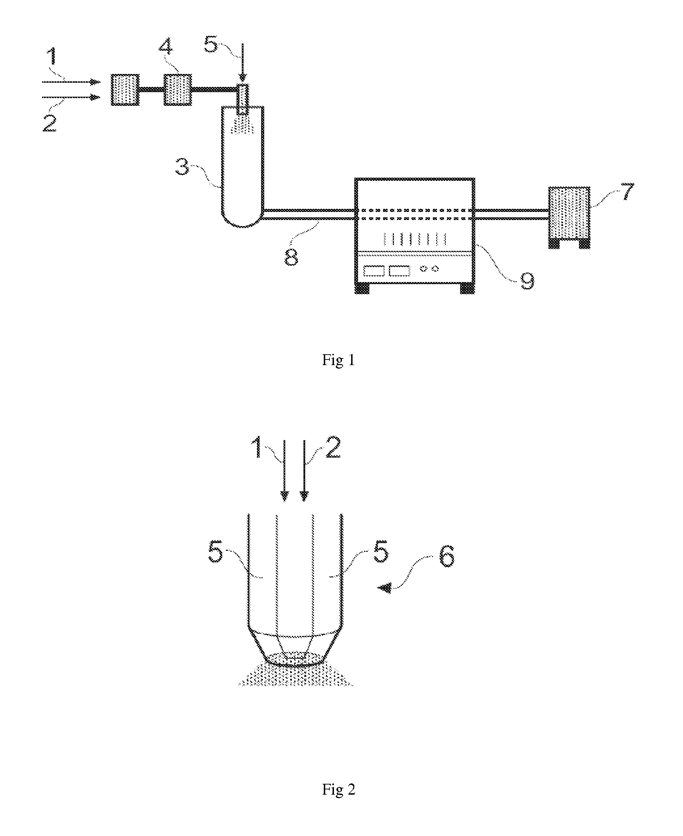

The particles of the invention may be made by any suitable known process, but are preferably made by an aerosol method. A suitable aerosol procedure is described with reference to Figure 2. In more detail, the encapsulating agent (1) and dye (2) are introduced in liquid form into a spray chamber (3), generally *via* means of a pump (4), together with a carrier gas (5) which is typically an inert gas such as nitrogen, or air dried by conventional methods for example. The liquid forms of the dye and encapsulating agent may be solutions, suspensions or dispersions, and are preferably both solutions. The liquid form of the encapsulating agent typically comprises at least one source or precursor of the siliceous encapsulating agent *per se* which, during the aerosol process, ultimately provides the desired siliceous encapsulating agent. The source of encapsulating agent may be considered to be a pre-polymer as, during aerosolisation, it will polymerise or crosslink to form the desired siliceous encapsulating agent. Preferably the siliceous precursor is organic. Suitable sources or precursors which may be used in the aerosol process to form particles of the invention include all those conventionally used in the art to form silica, silicates and zeolites, for example. Specific examples of useful silica precursors include tetramethylorthosilicate (TMOS), tetraethylorthosilicate (TEOS), tetrapropylorthosilicate (TPOS), tetraisopropylorthosilicate (TiPOS), tetrabutylorthosilicate (TBOS), silicic acid which may for example be modified with cations such as sodium or ammonium so that it is provided in the form of sodium silicate (also known as waterglass) or ammonium silicate. TEOS is a particularly preferred source of silica from a safety perspective, because the by-product of the process is ethanol (not methanol, as is the case for TMOS).

As is easily determinable and generally known by a skilled person, approximately one third of the weight of precursor is transformed into particles. For example, silica (SiO₂) has a molecular weight of 60g/mole and TEOS has a molecular weight of 208g/mole, so the weight of silica produced is 60/208 or 0.29 times the amount of TEOS. For TMOS the value is 0.39 (the molecular weight of TMOS is 152g/mole).

In addition, the amount of dye encapsulated within the particles of the invention is easily calculable by a skilled person. For example, if one wants a 10% dye loading, then, bearing in mind how much precursor one needs, as discussed above, it is a straightforward matter to calculate the amount of dye needed (a precursor solution of 10.4g of TEOS and 0.33g of dye, for example, yields silica particles comprising 90% silica (3g silica) and 10% dye (0.33g dye)).

The solvent used in the present invention will depend upon the hydrophobicity of the starting material. TEOS, TMOS and TPOS are hydrophobic and are therefore generally solubilised in an essentially non-aqueous material, for example as an alcoholic solution such as a solution in ethanol, methanol, n-propanol, iso-propanol and/or a butanol, ie. n-butanol, 2-butanol, iso-butanol or tert-butanol. Alternatively, a solution in acetone or one or more other conventional solvents, for instance, may also be employed. Silicic acid and the silicates are hydrophilic so may be dissolved in hydrophilic solvents such as water. The amounts of solvent used are readily determinable by a skilled person - the lower limit is, in practice, determined by the solubility parameters of the starting material and the upper limit is a practical one - the more solvent one uses, the smaller the final particles and the smaller the production capacity.

Although the solvent may be non-aqueous, some water is, nevertheless, necessary in order to hydrolyse the precursor, such as TEOS, to silicic acid prior to aerosolisation. Hydrolysis prior to aerosolisation is important to minimise the number of pores in then resulting particles, thereby minimising leakage of encapsulated dye. Typically, it is preferred that the aqueous portion be an acidic solution. The pH will be more than 1 and less than 7 and is advantageously approximately 2, as this is at or near the iso-electric point of silica itself. The pH of the precursor liquid form may be adjusted as desired using techniques conventionally used in the art, for example by addition of acid. A preferred acid used for this purpose is hydrochloric acid. Water of the requisite pH may be introduced as a solvent for the silica precursor or as a solvent for the dye, as discussed below.

The dyes incorporated into particles of the invention are provided to the aerosol process in acidic, basic or neutral liquid form. Preferably the one or more dyes is provided in the form of a solution in one or more solvents conventionally used in this field, preferably water or an alcohol such as methanol, ethanol, n-propanol, iso-propanol or a butanol (as previously defined), and particularly preferably ethanol or water. Highly preferably, the dye is provided in the form of an aqueous solution, and conveniently as an aqueous ethanolic solution. It may be necessary to aid dissolution of the active in the chosen solvent by providing it in the form of a salt, for instance those formed with commonly-used cations such as sodium, ammonium or potassium, or by adjusting the pH of the mixture of dye and solvent, again by conventional methods as previously described.

As mentioned above, it is desirable that, in creating the aerosol to form the particles of the invention, at least one of the liquid forms of encapsulating agent and dye should be aqueous. It is usually preferred that the dye be provided in aqueous liquid form, whereas the siliceous precursor is typically provided in non-aqueous form. The presence of water in the reaction medium aids pre-hydrolysis of the silica precursor which, in turn, aids subsequent polymerisation of the precursor to form the desired encapsulating agent comprising a low number of pores. The liquid forms of the dye and precursor are preferably mixed together prior to entry into the aerosol chamber for this purpose.

In addition and as mentioned above, if the siliceous encapsulating agent incorporates other inorganic materials in its structure in addition to silica, these may also be provided to the aerosol process in liquid forms of conventional sources of such materials. If it is desired to include titanium dioxide, for example, it may be appropriate to include a solution tetraethoxytitanate dissolved in an appropriate solvent, such as ethanol.

A suitable aerosol procedure is described with reference to Figures 1 and 2 in which the encapsulating agent (1) and dye (2) are mixed, then introduced in liquid form into a spray chamber (3), generally *via* means of a pump (4), together with a carrier gas (5) which is typically an inert gas such as nitrogen, or air dried by conventional methods for example.

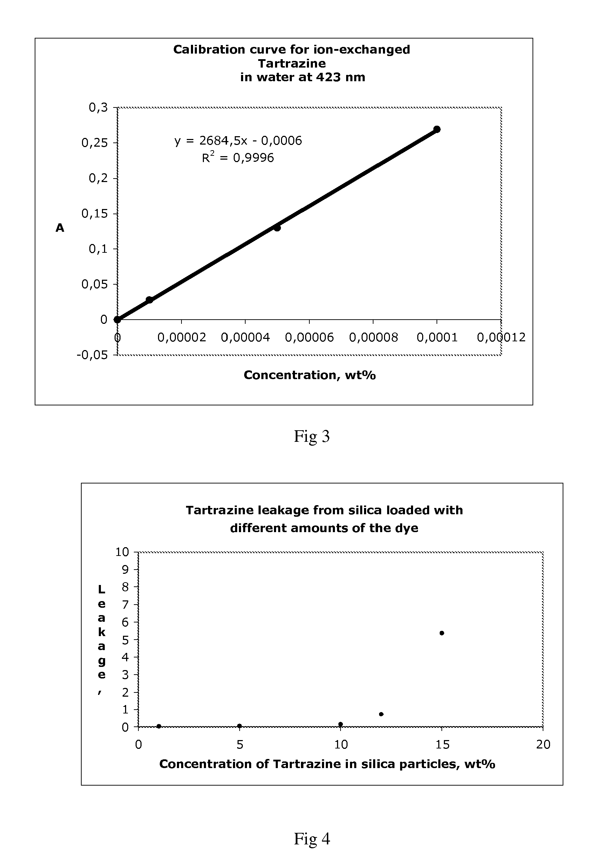

Typically a spray nozzle (6) such as that shown in Figures 1 and 2 is used in the aerosol process, whereby the dye (2) and agent (1) are introduced through a central tube and the carrier gas (5) is introduced through an outer tube of the nozzle. This type of nozzle is conventionally known as a "two-flow spray nozzle", however, other nozzle types commonly used in creating aerosols may also be employed. A two flow nozzle is preferred as the carrier gas flow cuts across or dissects the central flow of dye and encapsulating agent, thus facilitating more effective formation of spray droplets comprising the encapsulating agent and dye. Whilst the apparatus shown in Figures 2 and 3 illustrates a downwardly-spraying nozzle, it will be appreciated that all conventional types of aerosol apparatus including upwardly spraying apparatus may be conveniently used in the aerosol process. Indeed, so-called "spray up" systems may be preferred where it is desirable to fractionate particles of different sizes directly from the spray chamber, for instance.

The droplets formed in the spray chamber (3) are typically held in the chamber for a residence time in the range of greater than 0 up to about three minutes. Residence time may affect the porosity and, to a limited extent, the size of the resulting particles. For instance, for an average particle size of approximately 3-5 µm and a minimum porosity, a residence time of approximately 10 seconds may be conveniently employed. When present in the spray chamber, the encapsulating agent undergoes crosslinking within itself, thus forming droplets of a secure cage-like structure or network within which the dye is securely held. In addition, of course, the solvents evaporate. Typically the particles according to the invention have a diameter which is half that of the droplets sprayed into the spray chamber (3).

The droplets are then removed from the spray chamber in a conventional manner for instance *via* means of a pressure differential created by a pump (7) located at the end of a tube (8), into which the droplets pass from the spray chamber. Generally, the tube (8) into which the droplets pass is heated to a temperature which will effect drying of the particles for instance *via* means of a heater (9). Typically, a temperature in the range of approximately 150-250 °C is employed. Heating of the droplets in this way promotes condensation and, thus, further crosslinking of the siliceous encapsulating agent, preferably ultimately resulting in the formation of substantially fully crosslinked polymer-encapsulated dye particles.

The particles made in the aerosol process are typically dried by any means conventionally known in the art, such as a heater, either before or after their recovery from the aerosol apparatus which is, again, achieved in a conventional manner.

Optionally, the particles may undergo a subsequent washing process , if so desired, in order to ensure that all of the dye is securely encapsulated within the particles and that none remains at the surface of the particles following the process of the invention, for example. Conventional washing agents or solvents such as water, alcohols or acetone may be used for this purpose, the choice of washing agent typically being dependent upon the solubility characteristics of the relevant dye(s).

The conditions under which particles of the invention are produced by the aerosol process are not critical. Accordingly, aerosolisation may be performed under temperature, pressure and other conditions as desired by the skilled person in this technical field. Typically and conveniently, however, aerosolisation is performed under ambient temperature and pressure conditions, ie. at room temperature of approximately 18-25°C, and at a pressure of approximately atmospheric pressure. However, it will be appreciated that lower or higher temperatures and pressures may be employed as desired. In addition, it is not essential to exclude humidity from the aerosol apparatus. As such, the relative humidity (RH) within the aerosol apparatus does not need to be monitored but, under ambient conditions, is typically less than 50 %, as measured by conventional techniques.

Particles according to the present invention have a specific surface area of 0.1m²/g to 25m²/g, preferably 0.5m²/g to 5m²/g, more preferably 0.5m²/g to 3.5m²/g. In addition, particles according to the present invention have a specific internal pore volume of 0.001 to 0.03 cm³/g, preferably 0.001m³/g to 0.011cm³/g. Surface areas and pore volumes are determined using nitrogen porosimetry using nitrogen at a temperature of - 196°C or 77K. The samples are evacuated at 120-150°C for at least 4-6 hours to remove adsorbed water from the pores, and sample sizes are preferred to be around 0.5g. Otherwise standard procedures for collecting high quality N₂ isotherm data should be followed. The pore volumes are cumulative pore volumes for internal pores less than 50 nm in diameter and are determined using the "Barret-Joiner-Halenda" method.

The dye-loaded particles may be provided with a hydrophobic coating to improve the particles' dispersion in hydrophobic carrier medium. Advantageously, the hydrophobic coating may be made by applying a mixture of one or more of the following materials and isopropyl alcohol onto the dye-loaded powder and drying at 150°C for 3 hours: reactive organo-polysiloxane, polyolefin (including polyethylene and polypropylene), hydrogenated lecithin and salts thereof, N-acylamino acid and salts thereof and dextrin fatty acid esters. Preferably, the reactive organo-polysiloxane comprises organo hydrogen polysiloxane, triorgano siloxy silicic acid and organopolysiloxane modified at both terminal ends with trialkoxy groups. Commercially available materials falling into the category of reactive organo-polysiloxanes include KF-99, KF-9901, KF-7312F, KF-7312-J, KF-7312K, KF-9001, KF-9002, X-21-5249 and X-21-5250 manufactured by the Shin-Etsu Chemical Company Ltd; SH-1107, DC593, BY-11-015, BY-11-018 and BY-11-022 manufactured by Dow Corning Toray Silicone Co. Ltd.; TSF484, TSF483 and TSF4600 manufactured by Toshiba Silicone Co. Ltd.; FZ3704 and AZ6200 manufactured by Nippon Unicar Co. Ltd.

The hydrophobic coating is not limited to those described in the preceding paragraph and alternative hydrophobic coatings known to the skilled person may be employed instead. Such coatings may include trialkoyl isopropyl titanate, preferably triisostearoyl isopropyl titanate and perfluoro coatings, preferably polyperfluoroethoxymethoxy PEG-2 phosphate.

In addition to the hydrophobic coating, the dye-loaded particles may be provided with a coating of organo-functionalised silicone fibrils such as those described in EP 1602 352. Such fibril coatings may reduce or prevent agglomeration of dye-loaded particles.

Some coatings may both provide hydrophobic properties and exhibit fibrils to avoid flocculation. Commercially available coatings falling into this category include KF9908 (Triethoxysilylethyl Polydimethylsiloxyethyl Dimethicone), KF9909 (Triethoxysilylethyl Polydimethylsiloxyethyl Hexyl Dimethicone) and KP575 (Acrylate/Tridecyl Acrylate/Triethoxysilylproplyl Methacrylate/Dimethicone Methacrylate Copolymer) from the Shin Etsu Co Ltd.

The cosmetic compositions of the present invention may be in any suitable delivery form, such as emulsions, including oil-in-water, silicone-in-water, water-in-oil and water-in-silicone emulsions and multiple emulsions; anhydrous products, such as powders; gels; aerosols and mousses.

These delivery forms comprising the amorphous particles of the invention may be incorporated into a wide variety of products, including colour cosmetic compositions, such as make-up, foundation, nail varnish mascara, and lipstick; skin creams; skin cleansing products; body washes; hair cleansing products; hair conditioner; antiperspirant and deodorant products; fine fragrance products, such as eau de cologne, eau de toilette and eau de parfum.

The cosmetic compositions of the present invention may comprise cross-linked silicone elastomers, which elastomers may be emulsifying cross-linked organopolysiloxane elastomer, non-emulsifying cross-linked organopolysiloxane elastomer or mixtures thereof. As used herein, the term "non-emulsifying" when employed in relation to cross-linked organopolysiloxane elastomer includes cross-linked organopolysiloxane elastomer which comprise no polyoxyalkylene or polyglyceryl units. As used herein, the term "emulsifying" when employed in relation to cross-linked organopolysiloxane elastomer includes cross-linked organopolysiloxane elastomer which comprise at least one polyoxyalkylene (e.g., polyoxyethylene or polyoxypropylene) or polygyceryl unit.

Preferred non-emulsifying organopolysiloxane compositions are dimethicone/vinyl dimethicone crosspolymers. Such dimethicone/vinyl dimethicone crosspolymers are supplied by a variety of suppliers including Dow Corning (DC 9040 and DC 9041), General Electric (SFE 839), Shin Etsu (KSG-15, 16, 18 [dimethicone/phenyl vinyl dimethicone crosspolymer]), and Grant Industries (Gransil(TM) line of materials), and lauryl dimethicone/vinyl dimethicone crosspolymers supplied by Shin Etsu (e.g., KSG-31, KSG-32, KSG-41, KSG-42, KSG-43, and KSG-44).

Particularly useful emulsifying elastomers are polyoxyalkylene-modified elastomers formed from divinyl compounds, particularly siloxane polymers with at least two free vinyl groups, reacting with Si-H linkages on a polysiloxane backbone. Preferably, the elastomers are dimethyl polysiloxanes cross-linked by Si-H sites on a molecularly spherical MQ resin. Examples of commercially available emulsifying cross-linked organopolysiloxane elastomers include KSG-21 and KSG-210 and KSG-320 from the Shin-Etsu Chemical Company Ltd. Commercially available examples of emulsifying cross-linked organopolysiloxane elastomers comprising polyglyceryl units are KSG 710 and KSG-800 from the Shin-Etsu Chemical Company Ltd.

Cosmetic compositions according to the invention may comprise oil. The oil may be selected from the group consisting of volatile oils, non-volatile oils and mixtures thereof.

As used herein, the term "non-volatile" when employed in relation to an oil includes oils that fulfil at least one of the following definitions: (a) the oil exhibits a vapour pressure of no more than about 0.2 mm Hg at 25°C and one atmosphere pressure; (b) the oil has a boiling point at one atmosphere of at least about 300°C. As used herein, the term "volatile" when employed in relation to oils includes materials that are not "non-volatile" as previously defined herein.

Any non-volatile oil adhering to the above definition may be included in cosmetic compositions according to the invention. Such non-volatile oils may include silicone oils, both functionalised and non-functionalised, hydrocarbon oils and mixtures thereof.

Volatile oils which may be included in cosmetic compositions according to the invention may include silicone oils, both functionalised and non-functionalised, hydrocarbon oils and mixtures thereof. Volatile oil useful in the present invention may exhibit one or more of the following characteristics - it may be saturated or unsaturated, have a straight or branched chain or a cyclic structure. Examples of volatile hydrocarbons which may be incorporated into cosmetic compositions according to the invention include polydecanes such as isododecane and isodecane (e.g., Permethyl-99A which is available from Presperse Inc.) and the C₇-C₁₅ isoparaffins (such as the Isopar Series available from Exxon Chemicals). Examples of volatile silicone oils which may be incorporated into cosmetic compositions according to the invention include cyclic volatile silicones corresponding to the formula: 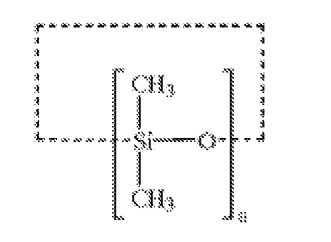 wherein n is from about 3 to about 7 and linear volatile silicones corresponding to the formula:

(CH₃)₃Si-O-[Si(CH₃)₂₋O]ₘ-Si(CH₃)₃

wherein m is from about 1 to about 20 preferably from 3 to 12.

Preferably, the cyclic volatile silicone is cyclopentasiloxane or cyclohexasiloxane. Linear volatile silicones generally have a viscosity of less than about 5 centistokes at 25°C; cyclic silicones generally have viscosities of less than about 10 centistokes at 25°C.

Examples of commercially available volatile silicone oils include the following cyclomethicones: Dow Corning 200, Dow Corning 244, Dow Corning 245, Dow Corning 344, and Dow Corning 345 (commercially available from Dow Corning Corp.); SF-1204 and SF-1202 Silicone Fluids (commercially available from G. E. Silicones), GE 7207 and 7158 (commercially available from General Electric Co.); and SWS-03314 (commercially available from SWS Silicones Corp.). Other examples of commercially available methyl silsesquioxanes available as TMF 1.5 fluid from Shin-Etsu Chemical Co; SILCARE SILICONES, for example phenyl substituted silsesquioxanes available as Silcare 15M60, n-Octyl substituted silsesquioxanes available as Silcare 31M60 and 31M50, hexyl methicone, caprylyl methicone and lauryl methicone available as Silcare 41M10, 41M15 and 41M20 respectively from Clariant.

In one advantageous embodiment, it is preferred that the volatile oil comprise a mixture of volatile cyclic silicone and volatile linear dimethicone of viscosity from 2 to 50 x 10⁻⁶m²/s (2-50cst), more preferably from 3 to 50 x 10⁻⁶m²/s (3-5cst), more preferably still from 3 to 50 x 10⁻⁶m²/s (4cst).

Preferred examples of linear dimethicones useful include DC200 5cst, DC1630 and DC 5-2117, More preferably, the linear dimethicone comprises DC 5-2117.

The cosmetic compositions of the present invention can also comprise a thickening agent, which may be a water phase thickening agent or an oil phase thickening agent.

Nonlimiting classes of water phase thickening agents comprise carboxylic acid polymers, crosslinked acrylate copolymers, polyacrylamide polymers or mixtures thereof:
(i) Carboxylic Acid Polymers These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Examples of carboxylic acid polymer thickeners useful herein are those selected from the group consisting of carbomers (available as the Carbopol 900^{™} series from B.F. Goodrich eg.Carbopol 954^{™}), acrylates/C10-C30 alkyl acrylate crosspolymers (commercially available as Carbopol 1342^{™}, Carbopol 1382^{™}, Pemulen TR-1^{™}, and Pemulen TR-2^{™}, from B.F. Goodrich) and mixtures thereof.
(ii) Crosslinked Acrylate Copolymers These polymers comprise a blend of a water soluble anionic acrylic monomer, a water soluble non-ionic acrylate monomer and a bifunctional monomeric cross-linking agent. Suitable water soluble anionic acrylic based monomers include acrylic acid, methacrylic acid and mixtures thereof. Suitable water-soluble non-ionic acrylate-based monomers include acrylamide, methacrylamide, N-vinyl pyrolidone, water-soluble hydroxy-substituted acrylic or methacrylic esters or mixtures thereof. Suitable bifunctional monomeric cross-linking agents include di, tri and tetraethylenically unsaturated materials such as methylene bis acrylamide, divinylpyrroline and allyl (meth) acrylate or mixtures thereof. Commercial examples of co-polymer compositions suitable for use herein include the co-polymer compositions commercially available from BASF Corp. under the tradename Luvigel ™ EM and the co-polymer compositions available from CIBA Speciality Chemicals, Macclesfield, UK, under the tradename Salcare SC91™.
(iii) Polyacrylamide Polymers Also useful herein are polyacrylamide polymers, especially anionic polyacrylamide polymers including substituted branched or unbranched polymers. These polymers can be formed from a variety of monomers including acrylamide and methacrylamide which are unsubstituted or substituted with one or two alkyl groups (preferably C₁ to C₅). Preferred are acrylate amide and methacrylate amide monomers in which the amide nitrogen is unsubstituted, or substituted with one or two C₁ to C₅ alkyl groups (preferably methyl, ethyl, or propyl), for example, acrylamide, methacrylamide, N-methacrylamide, N-methylmethacrylamide, N,N-dimethylmethacrylamide, N-isopropylacrylamide, N-isopropylmethacrylamide, and N,N-dimethylacrylamide. These polymers have a molecular weight greater than about 1,000,000 preferably greater than about 1,5000,000 and range up to about 30,000,000. Most preferred among these polyacrylamide polymers is the anionic polymer given the CTFA designation polyacrylamide and isoparaffin and laureth-7, available under the tradename Sepigel 305 from Seppic Corporation (Fairfield, NJ).

Suitable oil phase thickening agents can be selected from the group consisting of silicones, waxes, clays and mixtures thereof. Nonlimiting examples of these thickening agents are described below.

Suitable silicones include alkyl siloxane gellants, high molecular weight dimethicones (fluids greater than 1000 mPas), and high molecular weight alkyl, hydroxyl, carboxyl, amino, and/or fluoro- substituted dimethicones (fluids greater than 1000 mPas). Preferred silicone gellants are described in US patent 5,654,362 and 5,880,210, and include cyclomethicone and dimethicone crosspolymers (e.g., Dow Corning 9040).

Waxes can be defined as lower-melting organic mixtures or compounds of high molecular weight, solid at room temperature and generally similar in composition to fats and oils except that they contain no glycerides. Some are hydrocarbons, others are esters of fatty acids and alcohols. Suitable waxes may be selected from the group consisting of natural waxes including animal waxes, vegetable waxes, and mineral waxes, and synthetic waxes including petroleum waxes, ethylenic polymers, hydrocarbon waxes (e.g., Fischer-Tropsch waxes), ester waxes, silicone waxes, and mixtures thereof. Synthetic waxes include those disclosed in Warth, Chemistry and Technology of Waxes, Part 2, Reinhold Publishing (1956).

Specific examples of waxes include beeswax, lanolin wax, shellac wax, carnauba, candelilla, bayberry, jojoba esters, behenic acid waxes (e.g., glyceryl behenate which is available from Gattifosse as Compritol®), ozokerite, ceresin, paraffin, microcrystalline waxes, polyethylene homopolymers, polymers comprising ethylene oxide or ethylene (e.g., long chained polymers of ethylene oxide combined with a dihydric alcohol, namely polyoxyethylene glycol, such as Carbowax available from Carbide and Carbon Chemicals company; long-chained polymers of ethylene with OH or another stop length grouping at end of chain, including Fischer-Tropsch waxes as disclosed in Warth, supra, at pages 465-469 and specifically including Rosswax available from Ross Company and PT-0602 available from Astor Wax Company), C₂₄₋₄₅ alkyl methicones, C₈ to C₅₀ hydrocarbon waxes, alkylated polyvinyl pyrrolidones (e.g., "Ganex" alkylated polyvinylpyrrolidines available from the ISP Company) , fatty alcohols from C20 to C60 (e.g., "Unilins", available from Petrolite Corporation), and mixtures thereof.

Oil dispersible clays may be useful to provide structure or thickening. Suitable oil dispersible clays include organophilically modified bentonites, hectorites and attapulgites. Specific commercially available examples of these clays include Bentone 34 (Rheox Corp.) - Quaternium-18 Bentonite; Tixogel VP (United Catalysts) - Quaternium-18 Bentonite; Bentone 38 (Rheox Corp.) - Quaternium-18 Hectorite; Bentone SD-3 (Rheox Corp.) - Dihydrogenated Tallow Benzylmonium Hectorite; Bentone 27 (Rheox Corp.) - Stearalkonium Hectorite; Tixogel LG (United Catalysts) - Stearalkonium Bentonite; Claytone 34 (Southern Clay) Quaternium-18 Bentonite; Claytone 40 (Southern Clay) Quaternium-18 Bentonite; Claytone AF (Southern Clay) Stearalkonium Bentonite; Claytone APA (Southern Clay) Stearalkonium Bentonite; Claytone GR (Southern Clay) Quaternium-18/Benzalkonium Bentonite; Claytone HT (Southern Clay) Quaternium-18/Benzalkonium Bentonite; Claytone PS (Southern Clay) Quaternium-18/Benzalkonium Bentonite; Claytone XL (Southern Clay) Quaternium-18 Bentonite; and Vistrol 1265 (Cimbar) - Organophilic Attapulgite. These organophilic clays can be purchased as pre-dispersed organophilic clay in either an oil or an organic solvent. The materials are in the form of a heavy paste that can be readily dispersed into the formulation. Such materials include Mastergels by Rheox, United Catalysts, and Southern Clay.

Cosmetic compositions according to the present invention may additionally comprise an organic sunscreen. Suitable sunscreens may have UVA absorbing properties, UVB absorbing properties or a mixture thereof and include, but are not limited to, those found in the CTFA International Cosmetic Ingredient Dictionary and Handbook, 7th edition, volume 2 pp. 1672, edited by Wenninger and McEwen (The Cosmetic, Toiletry, and Fragrance Association, Inc., Washington, D. C., 1997). The exact amount of the sunscreen active will vary depending upon the desired Sun Protection Factor, i.e., the "SPF" of the composition as well as the desired level of UVA protection. The compositions of the present invention preferably comprise an SPF of at least 10, preferably at least 15. SPF is a commonly used measure of photoprotection of a sunscreen against erythema. The SPF is defined as a ratio of the ultraviolet energy required to produce minimal erythema on protected skin to that required to products the same minimal erythema on unprotected skin in the same individual (see Federal Register, 43, No 166, pp. 38206-38269, August 25, 1978).

Cosmetic compositions according to the invention may comprise metal oxide sunscreen particles. These particles may comprise any suitable metal oxide. Preferably, the metal oxide particles are selected from the group consisting of titanium oxide, zinc oxide, zirconium oxide, and cerium oxide. More preferably, the metal oxide particles are selected from titanium dioxide particles, zinc oxide particles or mixtures thereof. More preferably still, the metal oxide particles comprise titanium dioxide particles.

Advantageously, the metal oxide particles according to the invention have a number weighted average primary particle size from 10 to 100nm, preferably from 10-65nm, more preferably from 10 to 40nm and yet more preferable 10 to 25nm. As used herein, the term "primary particle size" means metal oxide crystal size, as determined by x-ray diffraction. In the case of titanium dioxide, it is based on measuring the broadening of the strongest rutile line.

Cosmetic compositions according to the invention may comprise other powders, in addition to amorphous particle and metal oxide sunscreen particles. Suitable powders include various organic and inorganic pigments that color the composition or skin. Organic pigments are generally various types including azo, indigoid, triphenylmethane, anthraquinone, and xanthine dyes which are designated as D&C and FD&C blues, browns, greens, oranges, reds, yellows, etc. Inorganic pigments are generally insoluble metallic salts of certified color additives, referred to as lakes or iron oxides. Suitable pigments include those generally recognized as safe, and listed in C.T.F.A. Cosmetic Ingredient Handbook, First Edition, Washington D.C. (1988). Specific examples are red iron oxide, yellow iron oxide, black iron oxide, brown iron oxide, ultramarine, FD&C Red, Nos. 2, 5, 6, 7, 10, 11, 12, 13, 30 and 34; FD&C Yellow No. 5, Red 3, 21, 27, 28, and 33 Aluminum Lakes, Yellow 5, 6, and 10 Aluminum Lakes, Orange 5 Aluminum Lake, Blue 1 Aluminum Lake, Red 6 Barium Lake, Red 7 Calcium Lake, and the like.

Other useful powder materials include talc, mica, titanated mica (mica coated with titanium dioxide), iron oxide titanated mica, magnesium carbonate, calcium carbonate, magnesium silicate, silica (including spherical silica, hydrated silica and silica beads), titanium dioxide, zinc oxide, nylon powder, polyethylene powder, ethylene acrylates copolymer powder, methacrylate powder, polystyrene powder, silk powder, crystalline cellulose, starch, bismuth oxychloride, guanine, kaolin, chalk, diatomaceous earth, microsponges, boron nitride and the like. Additional powders useful herein are described in US Patent No. 5,505,937.

Of the components useful as a matte finishing agents, low luster pigment, talc, polyethylene, hydrated silica, kaolin, titanium dioxide, titanated mica and mixtures thereof are preferred.

Micas, boron nitride and ethylene acrylates copolymer (e.g., EA-209 from Kobo) are preferred for imparting optical blurring effects through light diffraction and for improving skin feel, e.g., by providing a lubricious feel. Another particulate material for improving skin feel is SPCAT I2 (a mixture of talc, polyvinylidene copolymer, and isopropyl titanium triisostearate).

Preferred powders for absorbing oil are spherical, nonporous particles, more preferably having a particle size less than 25 microns. Examples of some preferred oil absorbing powders are Coslin C-100 (a spherical oil absorber commercially available from Englehard), Tospearl (spherical silica commercially available Kobo Industries), ethylene acrylates copolymer such as noted above, and SPCAT I2.

The powders may be surface treated with a hydrophobic and/or a fibril coating, as disclosed hereinabove.

The cosmetic compositions according to the invention may comprise one or more materials for imparting wear and/or transfer resistant properties, e.g., via film forming or substantive properties, may be used in the present compositions.

Such materials include film forming polymeric materials, such as:
a) sulfopolyester resins, such as AQ sulfopolyester resins, such as AQ29D, AQ35S, AQ38D, AQ38S, AQ48S, and AQ55S (available from Eastman Chemicals);
b) polyvinylacetate/polyvinyl alcohol polymers, such as Vinex resins available from Air Products, including Vinex 2034, Vinex 2144, and Vinex 2019;
c) acrylic resins, including water dispersible acrylic resins available from National Starch under the trade name "Dermacryl", including Dermacryl LT;
d) polyvinylpyrrolidones (PVP), including Luviskol K17, K30 and K90 (available from BASF), water soluble copolymers of PVP, including PVP/VA S-630 and W-735 and PVP/dimethylaminoethylmethacrylate Copolymers such as Copolymer 845 and Copolymer 937 available from ISP, as well as other PVP polymers disclosed by E.S. Barabas in the Encyclopedia of Polymer Science and Engineering, 2 Ed., Vol. 17, pp. 198-257;
e) high molecular weight silicones such as dimethicone and organic-substituted dimethicones, especially those with viscosities of greater than about 50,000 mPas;
f) high molecular weight hydrocarbon polymers with viscosities of greater than about 50,000 mPas;
g) organosiloxanes, including organosiloxane resins, fluid diorganopolysiloxane polymers and silicone ester waxes.
Preferred film forming polymers include organosiloxane resins comprising combinations of R₃SiO_{1/2} "M" units, R₂SiO "D" units, RSiO_{3/2} "T" units, SiO₂ "Q" units in ratios to each other that satisfy the relationship RₙSiO_{(4-n)/2} where n is a value between 1.0 and 1.50 and R is a methyl group. Note that a small amount, up to 5%, of silanol or alkoxy functionality may also be present in the resin structure as a result of processing. The organosiloxane resins must be solid at about 25°C and have a molecular weight range of from about 1,000 to about 10,000 grams/mole. The resin is soluble in organic solvents such as toluene, xylene, isoparaffins, and cyclosiloxanes or the volatile carrier, indicating that the resin is not sufficiently crosslinked such that the resin is insoluble in the volatile carrier. Particularly preferred are resins comprising repeating monofunctional or R₃SiO_{1/2} "M" units and the quadrafunctional or SiO₂ "Q" units, otherwise known as "MQ" resins as disclosed in U.S. Patent 5,330,747, Krzysik, issued July 19, 1994. In the present invention the ratio of the "M" to "Q" functional units is preferably about 0.7 and the value of n is 1.2. Organosiloxane resins such as these are commercially available such as Wacker 803 and 804 available from Wacker Silicones Corporation of Adrian Michigan, and G. E. 1170-002 from the General Electric Company.

Other materials for enhancing wear or transfer resistance include trimethylated silica. Suitable silicas of this type and cosmetic compositions containing them are described in US 5,800,816.

A variety of additional optional ingredients may be incorporated into the compositions of the present invention. Non-limiting examples of these additional ingredients include additional skin care actives such as precursors of the glycosaminoglycans, including, but not limited to acetylglucosamine and glucuronic acid; peptides (such as, palmitoyl-lys-thr-thr-lys-ser]), farnesol, bisabolol, phytantriol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin B₃ (e.g., niacinamide) and vitamin B₅ (e.g., panthenol) and the like and mixtures thereof; anti-acne medicaments (resorcinol, salicylic acid, and the like; antioxidants (e.g., phytosterols, lipoic acid); flavonoids (e.g., isoflavones, phytoestrogens); skin soothing and healing agents such as aloe vera extract, allantoin and the like; skin whitening agents, such as but not limited to, ascorbic acid and its derivatives, including sodium and magnesium ascorbyl phosphate; self-tanning agents, such as dihydroxyacetone; chelators and sequestrants; and agents suitable for aesthetic purposes such as essential oils, fragrances, skin sensates, opacifiers, aromatic compounds (e.g., clove oil, menthol, camphor, eucalyptus oil, and eugenol).

Humectants which may be included in cosmetic compositions according to the invention include polyhydric alcohols such as glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine and mixtures thereof. Most preferably the humectant comprises glycerine.

The compositions of the present invention may optionally include particulate materials. Particulate materials suitable herein include materials that are insoluble in both water and oil with a median particle size of from 1 to 50 µm,. Suitable particulate materials are organic or organosilicone or inorganic. Representative commercially available examples of useful particulate materials herein are Microthene FN510™, Tospearl 145™, Orgasol 2002™, Nylonpoly WL10™, Dry Flo™ or mixtures thereof.

### Examples

The present invention will now be described in more detail with reference to the following non-limiting example(s):

### Example 1: Preparation of Silica loaded with Tartrazine (FD&C Yellow No.5)

As a first step to synthesising sodium tartrazine-containing silica, the dye (commercially available from Sigma as T0388-100G (CAS# 1934-21-0) was ion-exchanged using a column with ion-exchanging resin (type Dowex 50Wx8 commercially available Dow Chemical Comp., Michigan,USA. This was necessary because the use of commercial Tartrazine induces flocculation of the tetraethylorthosilicate/ethanol/hydrochloric acid (TEOS/EtOH/HCl) encapsulating agent mixture.

### Column Preparation

The column was loaded with 317 g Dowex 50Wx8 to obtain a 400ml bed volume.
Step 1- Washing: to remove residual sodium cations, the column was eluted with 2.51 deionized water over 5 to 10 minutes at pH 6.
Step 2 - Reconditioning: to remove bound sodium cations the column was washed through with four batches of 400 ml 7% HCl. The contact time of HCl on the column was 45 minutes.
Step 3 - Washing: as for Step 1.
Step 4 - Charging: as for Step 2, but it was performed using 7% ammonium chloride (NH₄Cl) instead of HCl.
Step 5 - Washing: as for Step 1, the purpose being to remove excess ammonium cations.

### Ion Exchange of Dye

A 10% dye solution of sodium tartrazine was prepared in an acidic solution of water and ethanol and was eluted through the column. This solution was used to produce Tartrazine-containing silica in the subsequent procedure.

### Spraying of Silica with Yellow Dye

Two batches of coloured silica were each prepared using 10.4 g TEOS, 5.4 g of HCl with a pH of around 1.25 and 12.0 g of ethanol. The components were mixed together and the mixture was left stirring for 30 minutes. Theoretically, such a mixture should give 3 g of silica after aerosolisation. The calculation of the amount of Tartrazine solution to add was based on this theoretical amount of silica. The ion-exchanged Tartrazine solution was then mixed with 4 g of ethanol.

The two resulting mixtures were then blended together, the pH was adjusted to pH 2.0 using 1M HCl and the blend was left under stirring for a further 10 minutes. The blend was then aerosolized and spray-dried as follows:

The starting solution blend is pumped at a constant rate of 3ml/minute using a peristaltic pump to the centre flow outlet of a coaxial two-flow spray nozzle of a spray tower. At the same time, compressed air is pumped at 20litres/minute (at STP) to outer annular outlet located coaxially around the centre flow outlet. The centre flow outlet diameter is 1mm; the outer diameter is 1.5mm. The spraying was such that a turbulent mixture was propelled into the spray chamber, which was retained at ambient temperature. Afterwards, the mixture was heated to 220°C to induce cross-linking and drying of the particles.

### Washing of Particles

The particles were washed with de-ionized water, at a rate of 200 ml water per 1 g of particles as follows: 5 g of particles were placed into a plastic bottle and 1000 ml of water were added. The mixture was left under stirring for 5 minutes and it was then centrifuged for 10 minutes at 3500 rpm. The sediment was then separated from the supernatant fluid.

### Separation of Small Particles

The sediment from the centrifuged mixture was mixed with 1000 ml water in a beaker and left to settle for two days. The resulting supernatant fluid was then pumped into another beaker using a roll pump. Once the supernatant had been pumped into the other beaker, it was centrifuged at 3500 rpm for 20 minutes and the resulting sediment was separated using a standard separation technique from the liquid. The resulting particles were then dried in an oven at 50°C.

### Particle Size Measurements

The size of the resulting particles was measured using a Malvern Master Sizer 2000 apparatus, which measures particle size *via* light scattering Particle size distribution is as follows:
d(0.1): 0.308µm (10% of the particles have a size lower than the volume averaged value given)
d(0.5): 0.539µm (50% of the particles have a size lower than the volume averaged value given)
d(0.9): 0.953µm (90% of the particles have a size lower than the volume averaged value given)

### Example 2: Preparation of Silica loaded with Amaranth (Acid Red No.27)

This red dye is soluble in water but it is not soluble in ethanol. It was not necessary to ionexchange the aqueous solution (as was done for Tartrazine in Example 1), however, as it did not flocculate when it was blended with the TEOS mixture. Also, even though the dye was insoluble in ethanol, it was still possible to use it directly by increasing the water proportion in the aerosol mixture.

### Sample Preparation

Two batches of coloured silica were prepared using 10.4 g TEOS, 5.4g of HCl of pH 2 and 12.0 g of ethanol. The components were mixed together and the mixture was left stirring for 30 minutes. Theoretically, the mixture would give 3 g of silica after the aerosolisation. Thus, the calculation of the amount of Amaranth solution (obtained from Sigma has catalogue number A1016-100G (CAS#915-67-3)) to add was based on this amount of silica. This was as follows: 0.3 g of Amaranth powder plus 10.0 g of HCl (pH 2). The two mixtures were mixed together and left to stir for 10 minutes. The mixture was subsequently spray dried, heated, washed, the particles separated and size measured as in example 1. Particle size distribution is as follows:
d(0.1): 0.322µm
d(0.5): 0.592µm
d(0.9): 1.130µm

### Example 3: Preparation of Silica loaded with Erioglaucine (FD & C Blue No. 1)

This blue dye is soluble in water and ethanol and it was not necessary to ion exchange the solution.

### Sample Preparation

Two batches of coloured silica were prepared each using 10.4 g TEOS, 5.4 g HCl (pH2) and 8.0 g of ethanol. The components were mixed together and the mixture was left under stirring for 30 minutes. Theoretically, the mixture would give 3g of silica after the aerosolisation. Thus, the calculation of the amount of Erioglaucine solution to add was based on this amount of silica. This was as follows: 0.3 g of Erioglaucine powder (obtained from Sigma/Aldrich, catalogue# 861146-25G (CAS#3844-45-9)) plus 2.0 g of HCl (pH 2) in 3 g ethanol. The two mixtures were mixed together and left to stir for 10minutes. The mixture was subsequently spray dried, heated, washed, the particles separated and size measured as in example 1. Particle size distribution is as follows:
d(0.1): 0.327 µm
d(0.5): 0.59 µm
d(0.9): 1.130 µm

### Example 4: Dye Release/Leakage Experiments

### Example 4A

For the products of each of Examples 1, 2 and 3, 0.2 g of the particles loaded with dye were placed into a centrifuge tube and 10 ml of a water/propanol (1:1) mixture was added. The tube was shaken for two minutes and centrifuged. The supernatant fluid was separated from the sediment and collected in a bottle. This operation was repeated five times. The supernatant fluid from all five extractions was mixed and analysed using a UV spectrometer The results are provided in the following table (3).

| **Table 3** | | | |
|---|---|---|---|
| Wash number | Release Yellow, wt% | Release Red, wt% | Release Blue, wt% |
| 1 | 0.25 | 0.36 | 0.1299 |
| 2 | 0 | 0.027 | 0.0196 |
| 3 | 0 | 0 | 0.0091 |
| 4 | 0 | 0 | 0 |
| 5 | 0 | 0 | 0 |
| Sum over five washes | 0.25 | 0.387 | 0.1586 |

The results show that the release of the dyes into the water/propanol mixture was extremely low.

### Example 4B

The leakage of Tartrazine from silica loaded with different amounts of dye was investigated as follows. In turn, 1 g of particles loaded with 1%, 5%, 10%, 12% and 15% respectively of Tartrazine was placed into a bottle and 100 g of water was added. The mixture was left under stirring for 3 hours, after which time, a 5 ml portion was extracted from every bottle using a syringe. This portion was filtered with a membrane filter (0.45 µm) and analyzed in an UV-VIS spectrophotometer. The leakage in wt% was calculated for every sample with help of the calibration curve presented as Figure 3, which shows the calibration for ion-exchanged Tartrazine in water at 423 nm.

The results are presented in the following table (4).

| **Table 4** | |
|---|---|
| **Tartrazine Concentration (wt%)** | **Leakage (wt%)** |
| 1 | 0.044 |
| 5 | 0.0528 |
| 10 | 0.155 |
| 12 | 0.727 |
| 15 | 5.37 |

These results are presented in Figure 4, and show that dye leakage, in the case of Tartrazine-loaded particles, substantially increases at greater than 12 wt% Tartrazine concentration within the particles.

### Example 5

A lipstick according to the invention and having the following composition was prepared:

| **Ingredient** | **Wt%** |
|---|---|
| Carnauba | 1.50 |
| Ozokerite | 5.50 |
| Candelilla | 4.00 |
| Hydrogenated Vegetable Oil | 8.50 |
| Acetylated Lanolin | 4.00 |
| Propylparaben | 0.10 |
| Cetyl Ricinoleate | 10.00 |
| Ascorbyl Palmitate | 1.00 |
| Polybutene | 2.00 |
| Polysiloxane Copolymer¹ | 5.50 |
| Stearyl Dimethicone (DC 2503 Cosmetic wax) | 5.50 |
| Anhydrous Lanolin | 5.80 |
| DC 9040² Elastomer gel | 20.00 |

| **Association Structure Phase** | |
|---|---|
| Lecithin | 5.00 |
| Niacinamide | 2.50 |
| Panthenol | 0.1 |
| Glycerine | 4.00 |
| Encapsulated FD&C Red 40 | 9.00 |
| Water | 6.00 |

| | |
|---|---|
| ¹ #1154-141-1, supplied by GE Silicones. ² 13% Dimethicone/vinyl dimethicone crosspolymer in cyclomethicone. | |

The ingredients for the Association Structure Phase, except for the pigments, are mixed until association structures are formed. Once the association structures are formed, the pigments are added and milled on a three-roll mill. The mixture is then mixed with the remaining ingredients and mixed until a homogeneous mixture is achieved. (Or, alternatively, the above components are added and mixed together at the same time.) This mixture with mixing is heated to 85°C and then poured into a mold at room temperature.

The lipstick is applied to the lips to provide color, moisturization and improved lip feel.

### Example 6

A mascara according to the invention and having the following composition was prepared:

| **Ingredient** | **Wt.%** |
|---|---|
| Carnauba Wax | 3.00 |
| Glyceryl Monostearate¹ | 7.50 |
| White Beeswax | 3.75 |
| C18-C36 Triglycerides² | 5.50 |
| Hydrogenated Glycerol Rosinate³ | 0.15 |
| Propylparaben | 0.10 |
| Paraffin Wax 118/125 | 2.25 |
| Paraffin Wax | 2.25 |
| Elastomer Gel (DC9040) ⁴ | 17.31 |
| Lecithin⁵ | 2.25 |
| Stearic Acid 3X | 4.00 |
| Oleic Acid | 0.75 |
| Triethanolamine | 1.25 |
| Potassium Cetyl Phosphate⁶ | 1.00 |
| Shellac, NF | 3.00 |
| Triethanolamine | 0.47 |
| Trisodium EDTA | 0.10 |
| Encapsulated D&C Brown 1 | 7.00 |
| Simethicone | 0.20 |
| Methylparaben | 0.20 |
| Ethylparaben | 0.15 |
| Phenoxyethanol | 0.80 |
| Ethyl Alcohol 40B, 190 proof | 4.00 |
| Diazolidinyl Urea | 0.20 |
| Deionized Water | 30.22 |
| Dl-Panthenol | 0.35 |
| Niacinamide | 2.25 |
| Total | 100.00 |

| | |
|---|---|
| ¹ Available as Emerest 2400 available form Henkel/Emery ² Available as Syncrowax HGL-C available from Croda, Inc. ³ Available as Foral 105 available from Hercules, Inc. ⁴ 13% Dimethicone/vinyl dimethicone cross-polymer in cyclomethicone ⁵ Available as Centrolex F available from Central Soya, Inc. ⁶ Available as Amphisol K available from Givaudan | |

The waxes and fats are mixed in a vessel equipped with a heating source. The waxes and fats are heated and mixed at low speed using a conventional blender to liquefy the mixture. The mixing is continued until the mixture is homogeneous. To the homogenous mixture is added the pigments. The mixing rate is increased to high and the pigments are mixed into the mixture for about 30-35 minutes until uniformly dispersed. The mixing is continued while adding emulsifiers.

In a second vessel equipped with a heating source is added water followed by the niacinamide, lecithin and any other water-dispersible components. The mixture is heated and mixed to a temperature of from about 80-95°C. Additional water is added as necessary to account for water loss.

The aqueous and lipophilic mixtures are combined and mixed using a dispersator type mixer. Mixing is continued until the mixture cools to a temperature of from about 65-70°C. Preservatives are added with mixing, allowing the mixture to cool further to 45-47°C. Any remaining components are added with mixing. The combined mixture is cooled to a temperature above the solidification point and is then poured into suitable containers.

The mascara composition is applied to the lashes and/or eyebrows to provide softening, moisturization and conditioning.

### Example 7

A liquid foundation according to the invention and having the following composition was prepared:

| **Example #** | **3** | **4** |
|---|---|---|
| **Ingredient** | **% w/w** | **% w/w** |
| DC9040 cross linked elastomer gel¹ | 25.00 | 20.00 |
| Dimethicone copolyol cross-polymer (KSG21)² | ----- | 5.00 |
| Cyclomethicone | 10.00 | 5.00 |
| PEG/PPG18/18 Dimethicone & Cyclomethicone (DC5185) ¹ | 2.0 | 2.0 |
| Octyl Methoxy cinnamate | ----- | 4.00 |
| Diethylhexyl carbonate (Tegosoft DEC) | | 4.00 |
| 4cst Dimethicone (DC5-2117) | 4.00 | ----- |
| Methicone coated TiO₂ | 8.0 | ----- |
| Hydrophobic Encapsulated FD&CYellow5 | 3.3 | ----- |
| Hydrophobic Encapsulated FD&C Red 40 | 3.0 | ----- |
| Hydrophobic Encapsulated FD&C Blue 1 | 0.1 | ----- |
| TiO₂ | ----- | 10 |
| Encapsulated FD&C Yellow 5 particles | ----- | 5.0 |
| Encapsulated FD&C Red 40 particles | ----- | 4.0 |
| Encapsulated FD&C Blue 1 particles | ----- | 0.5 |
| Propylparabens | 0.1 | 0.1 |
| Ethylparabens | 0.1 | 0.1 |
| Methylparabens | 0.1 | 0.1 |
| Disodium EDTA | 0.1 | 0.1 |
| Benzyl alcohol | 0.5 | 0.5 |
| Sodium chloride | 2.00 | 2.00 |
| Glycerin | 10.00 | 12.00 |
| Niacinamide | 2.00 | 5.00 |
| Water | qs | qs |

| | | |
|---|---|---|
| ¹⁻ available from Dow Coming ²⁻ available from Shin Etsu Silicones | | |

In a suitable vessel, water, glycerine, disodium EDTA and benzyl alcohol are added and mixed using conventional technology until a clear water phase is achieved. When the water phase is clear, the methylparabens are added and mixed again until clear. Encapsulated pigments which are not hydrophobic are then added at this stage. The resultant phase is mixed with a Silverson SL2T or similar equipment on high speed (8,000 rpm, standard head). In a separate vessel, the KSG21, DC245, hydrophobic pigment encapsulates, other oils and the parabens are added and the mixture is milled using a Silverson SL2T on a high speed setting until a homogeneous mixture is created.

Following this step, the water phase and the silicone phase are combined and milled using the Silverson SL2T on a high speed setting until the water is fully incorporated and an emulsion is formed. The elastomer is then added and the mixture is mixed again using the Silverson on a high speed setting to generate the final product.

### Example 8

A skin cream according to the invention and having the following composition was prepared:

| **Example #** | **5** | **6** |
|---|---|---|
| **Ingredient** | **% w/w** | **% w/w** |
| *Deionised water* | QS | QS |
| Disodium EDTA | 0.1 | 0.1 |
| Glycerin | 10.0 | 7.0 |
| Niacinamide | 3.5 | 2.0 |
| Panthenol | 1.0 | 0.25 |
| Emulgade ¹ | 0.2 | 0.2 |
| Isohexadecane | 3.0 | 0.0 |
| Ethyl parabens | 0.15 | 0.15 |
| Propyl parabens | 0.07 | 0.07 |
| Stearic acid | 0.1 | 0.1 |
| PEG-100 Stearate ² | 0.1 | 0.1 |
| Stearyl alcohol | 0.60 | 0.4 |
| Cetyl alcohol | 0.50 | 0.6 |
| Behenyl alcohol | 0.40 | ----- |
| Isopropyl isostearate ³ | 1.5 | 3.0 |
| DL-α Tocopherol acetate | 0.5 | 0.1 |
| Petrolatum | 2.0 | ----- |
| Luvigel EM ⁴ | 2.0 | ----- |
| Sepigel 305 ⁵ | ----- | 2.0 |
| Sodium hydroxide | 0.011 | 0.011 |
| GLW75-PFAP MAP TiO₂ dispersion⁶ | 8.0 | 2.0 |
| Encapsulated FD&C Yellow 5 particles | 3.0 | 0.7 |
| Encapsulated FD&C Red 40 particles | 1.0 | 0.3 |
| Encapsulated FD&C Blue 1 particles | 0.1 | 0.1 |
| Microthene FN510⁷ | 1.0 | 1.0 |
| DryFlo Plus ⁸ | 1.0 | 1.5 |
| Benzyl alcohol | 0.25 | 0.25 |
| DC 1503 ⁹ | 1.0 | 1.5 |
| Perfume | 0.3 | 0.1 |

| | | |
|---|---|---|
| 1. Emulgade : Available from Cognis Deutchland GmbH 2. PEG 100 Stearate supplied by Uniqema 3. Supplied by Scher Chemicals Inc, Industrial West, Clifton, NJ 07012 4. Luvigel EM available from BASF GmBH 5. Sepigel 305 as supplied by Seppic. 6. GLW75-PFAP-MP as supplied by Kobo Chemicals. 7. Microthene supplied by Equistar Chemicals. 8. Dry Flo supplied by National Starch Chemical Company 9. DC 1503 supplied by Dow Coming. | | |

A water phase is prepared by admixing all water soluble ingredients, except sodium hydroxide, in water and heating to about 80°C. A second premix is prepared by admixing of the oil soluble ingredients except the silicone oil (DC1503) and heating also to around 80°C. The oil phase is added to the water phase and sheared to form an emulsion.

The emulsion is cooled to 60°C and the polymeric thickener (Luvigel EM or Sepigel 305) is then added. Sodium hydroxide solution is then added to neutralise to pH 6-7.5. At 45-50°C the benzyl alcohol, DC 1503, dyes and particles (including encapsulated pigments) are added and the resulting product is sheared to ensure particle dispersion, de-agglomeration and homogeneity. The composition can then be cooled to 40°C and perfume can be added. The product can then be prepared for packaging.

### Example 9

A tinting shampoo according to the invention and having the following composition was prepared:

| **Ingredient** | **Wt%** |
|---|---|
| Sodium Laureth Sulfate | 10.00 |
| Sodium Lauryl Sulfate | 6.00 |
| Polyquaterium-10² | 0.40 |
| Dimethicone³ | 0.50 |
| Ethylene Glycol Distearate | 1.50 |
| Cocamide MEA | 1.50 |
| 5-Chloro-2-methyl-4-isothiazolin-3-one, Kathon CG | 0.0005 |
| Sodium Benzoate | 0.25 |
| Disodium EDTA | 0.13 |
| Perfume | 0.70 |
| Encapsulated FD&C Red 40 particles | 0.01 |
| Citric Acid/ Sodium Citrate Dihydrate | pH QS |
| Sodium Chloride/ Ammonium Xylene Sulfonate | Visc. QS |
| Water | QS |

| | |
|---|---|
| ¹N-Hance 3269 (with Mol.W of about 500,000 and 0.8 meq/g) available from Aqualon/Hercules ²Polymer LR30M available from Amerchol/Dow Chemical ³Viscasil 330M available from GE Silicones | |

The shampoo may be prepared using conventional formulation and mixing techniques. Melting or dissolution of solid surfactants can be achieved by adding these to a premix of the surfactants, or some portion of the surfactants, mixed and heated to melt the solid components, e.g., about 72°C. This mixture can then optionally be processed through a high shear mill and cooled, and then the remaining components are mixed in. The viscosity of the composition is adjusted by adding the sodium chloride and ammonium xylene sulphonate.

### Example 10

A leave on hair conditioner according to the invention and having the following composition was prepared:

| **Component** | **Wt%** |
|---|---|
| Water | QS |
| Encapsulated FD&C Red 40 particles | 2.000 |
| L-glutamic Acid | 0.640 |
| Stearamidoprpyldimethylamine (SAPDMA) | 2.30 |
| Cetyl Alcohol | 2.50 |
| Stearyl Alcohol | 4.50 |
| Dimethicone/Cyclomethicone (15/85 blend) | 4.20 |
| EDTA | 0.100 |
| Benzyl Alcohol | 0.400 |
| Kathon CG | 0.0005 |
| DL Pantyl | 0.050 |
| DL-Panthenol | 0.050 |

In a suitable mixing vessel, form a lamellar gel matrix as follows: obtain deionized water at a temperature of about 85°C, and add Stearamidopropyldimethylamine, Cetyl Alcohol, Stearyl Alcohol, and L-glutamic Acid. Maintain the mixture at a temperature of about 85°C for 5 minutes, such that the ingredients are homogenized and no solids are observed. Cool the mixture to about 55°C, and maintain at this temperature until a lamellar gel matrix forms. Add encapsulated dye and mix for about 15 minutes at a temperature of about 35°C. Add the remaining ingredients also to the lamellar gel matrix.

## Claims

1. A cosmetic composition comprising:
(a) amorphous particles, each amorphous particle comprising a homogeneous distribution of one or more dyes encapsulated by an amorphous, siliceous encapsulating agent, wherein the amorphous particle comprises from 3% to 20%, preferably 5% to 15%, more preferably 8% to 12% dye, by weight of the particle;
(b) a cosmetically acceptable carrier.

2. The cosmetic composition according to claim 1, wherein the encapsulating agent is silica.

3. The cosmetic composition according to claim 1 or 2, wherein the one or more dyes is cationic.

4. The cosmetic composition according to any preceding claim, wherein the or each dye is xanthene, triarylmethane, anthracene, monoazo dye or a mixture thereof.

5. The cosmetic composition according to any preceding claim, wherein the amorphous particles have an average particle size of greater than 0 to 10 µm, preferably greater than 0 to 5 µm, more preferably from 10 nm to less than 1µm.

6. The cosmetic composition according to any preceding claim, wherein the amorphous particles have a specific surface area of 0.5m²/g to 5m²/g, preferably 0.5m²/g to 3.5m²/g.

7. The cosmetic composition according to any preceding claim, wherein the amorphous particles have a specific internal pore volume of 0.001 to 0.03 cm³/g, preferably 0.001m³/g to 0.011cm³/g.

8. The cosmetic composition according to any preceding claim, wherein the amorphous particles are spherical.

9. The cosmetic composition according to any preceding claim, wherein the amorphous particles are coated with a hydrophobic coating.

10. The cosmetic composition according to any preceding claim, wherein the cosmetically acceptable carrier comprises a silicone elastomer.

11. The cosmetic composition according to any preceding claim, wherein the cosmetically acceptable carrier comprises an organosiloxane resin.

12. The cosmetic composition according to any preceding claim, wherein the cosmetically acceptable carrier comprises a wax.

13. The cosmetic composition according to any preceding claim, wherein the cosmetically acceptable carrier comprises additional particles, the additional particles comprising metal oxide sunscreen particles, inorganic pigment particles, organic pigment particles or mixtures thereof.

14. The cosmetic composition according to any preceding claim, which is a make-up, a foundation, a nail varnish, a mascara, or a lipstick.

15. The cosmetic composition according to any of claims 1 to 13, which is, a skin cream, a skin cleanser, a body wash, a hair shampoo, a hair conditioner, an antiperspirant, a deodorant, an eau de cologne, an eau de toilette or an eau de parfum.
